# EUROPÄISCHE PATENTSCHRIFT

(11) **EP 0 775 124 B1**
(45) Veröffentlichungstag und Bekanntmachung des Hinweises auf die Patenterteilung: **19.09.2001**
(21) Anmeldenummer: 95928489.4
(22) Anmeldetag: 29.07.1995
(51) Int. Cl.: C07D 275/06, A01N 43/80, C07B 41/08

(54) **HERBIZIDE SACCHARINCARBONSÄURE-DERIVATE UND VERFAHREN ZU IHRER HERSTELLUNG**
HERBICIDAL SACCHARIN CARBOXYLIC ACID DERIVATIVES AND METHOD FOR THEIR PREPARATION
DERIVES D'ACIDE CARBOXYLIQUE DE SACCHARINE HERBICIDES ET LEUR PROCEDE DE FABRICATION

(30) Priorität: 08.08.1994 DE 4427996
(43) Veröffentlichungstag der Anmeldung: 28.05.1997
(73) Patentinhaber: BASF AKTIENGESELLSCHAFT, 67056 Ludwigshafen (DE)
(72) Erfinder: PLATH, Peter, D-67227 Frankenthal (DE); RANG, Harald, D-67122 Altrip (DE); WESTPHALEN, Karl-Otto, D-67346 Speyer (DE); GERBER, Matthias, D-67117 Limburgerhof (DE); WALTER, Helmut, D-67283 Obrigheim (DE)
(86) Internationale Anmeldenummer: EP9503022
(87) Internationale Veröffentlichungsnummer: WO9605184

(56) Entgegenhaltungen:
- EP-A- 0 308 371
- WO-A-89/10921
- FR-A- 2 525 593
- US-A- 3 673 200
- US-A- 4 410 353
- US-A- 5 034 534

## Beschreibung

Die vorliegende Erfindung betrifft die Herstellung von Saccharincarbonsäuren und Saccharincarbonsäureestern der Formel I 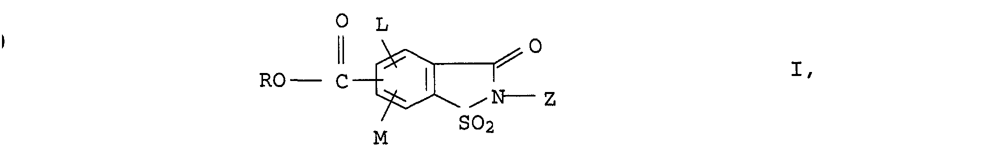 in der die Substituenten folgende Bedeutung haben:
- L, M: Wasserstoff, C₁-C₄-Alkyl;
- Z: Wasserstoff, C₁-C₄-Alkyl;
- R: H, C₁-C₆-Alkyl,
mit der Maßgabe, daß Z nicht Methyl, Wasserstoff oder ein Alkalimetall- oder Silberkation bedeuten, wenn L und M für Wasserstoff stehen, sowie ferner ausgenommen 5-Carboxy-7-methylsaccharin.

Ferner betrifft die Erfindung ausgewählte Saccharinderivate I, die eine herbizide Wirksamkeit aufweisen und als Zwischenprodukte für die Herstellung von Saccharinderivaten dienen, in denen der Rest OR durch andere Gruppen ersetzt ist. Diese Folgeprodukte sind Gegenstand von zeitgleichen deutschen Anmeldungen.

Gegenstand der Erfindung ist weiterhin ein Verfahren zur Bekämpfung unerwünschten Pflanzenwuchses mit den Verbindungen Ia.

Gemäß dem Stand der Technik, z.B. DE-A 36 07 343 werden Saccharinderivate mit einem Carboxysubstituenten im Phenylring über folgende Reaktionssequenz erhalten:

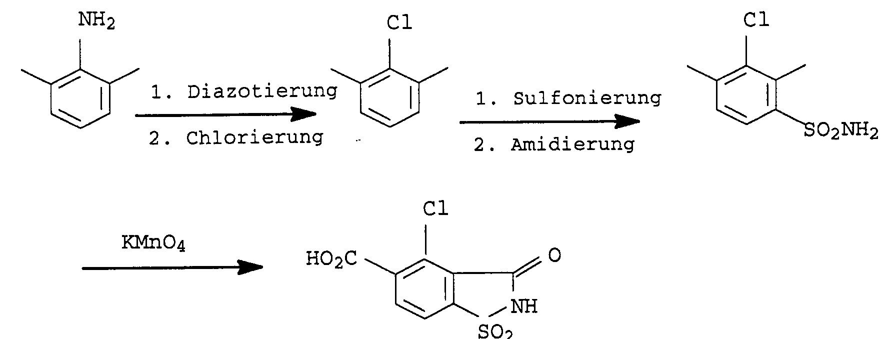

D.h. die Carboxyfunktion wird durch Oxidation einer Methylgruppe im Vorprodukt eingeführt, wobei gleichzeitig oxidativer Ringschluß erfolgt (s. auch deutsches Reichspatent 671 788 aus 1936) Diese Methode ist insofern nachteilig, als bei Vorhandensein mehrerer oxidierbarer funktioneller Gruppen keine selektive Oxidation gewährleistet ist. Zudem ist die Stufenzahl des Gesamtverfahrens sehr hoch, was zwangsläufig mit einer Ausbeuteverminderung verbunden ist.

In der US 4,410,353 und der FR-A 2 525 593 werden Saccharin-Derivate beschrieben, die am Stickstoff durch ein substituiertes Phenoxybenzamids substituiert sind.

Aus der US 3,673,200 sind Saccharin-Derivate bekannt, die über den Sauerstoff der Enolform an eine Phenyl- oder Pyridyl-Gruppe gebunden sind.

Die WO 89/10921 beschreibt herbizide Sulfonylamide, bei deren Herstellung als Nebenreaktion durch eine Cyclisierung Saccharin Derivate entstehen können.

Die EP-A 0 308 371 beschreibt die Herstellung von 4-Azasaccharinen, 4-Azadihydro- oder -tetrahydrosaccharinen aus Azadienen und Isothiazolin-3-on-1,1-dioxid-Derivate.

Die EP-A 0 158 074 beschreibt die Verwendung von Amin-Salzen des Saccharins als fungizide und bakterizide Mittel.

Benicha, Azmani, Akssira und Hurst beschreiben in Aust. J. Chem., 1993, 46, 903-906 ein Verfahren zur Herstellung von Arylsulfonylharnstoff-Derivaten des Saccharins.

Im US-Patent 5,034,534 wird die Herstellung von Saccharinderivaten durch Carbonylierung von chlorierten aromatischen Sulfonamiden in Gegenwart von Komplexen aus Palladium und mindestens einem Alkylphosphinliganden beschrieben. Über einer Einführung der Carboxylfunktion in den Phenylring des Saccharingerüstes ist der Lehre dieses Dokumentes nichts zu entnehmen.

Der Erfindung lag nun die Aufgabe zugrunde, den Zugang zu Saccharinderivaten der Formel I mit einer Carboxylfunktion im Phenylring zu ermöglichen, wobei drastische oxidative Methoden, wie die Verwendung von Kaliumpermanganat zu vermeiden waren.

Es wurde nun ein Verfahren zur Herstellung von Saccharinderivaten der Formel I gefunden, das dadurch gekennzeichnet ist, daß man entsprechende brom- oder iodsubstituierte Saccharinderivate der Formel II 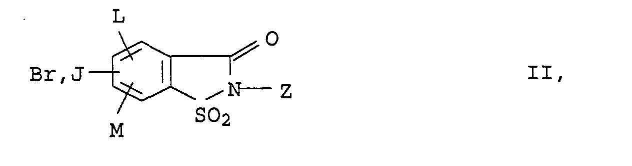 in der L, M und Z die obengenannte Bedeutung haben, oder im Falle von Z ≠ H Verbindungen der Formel III 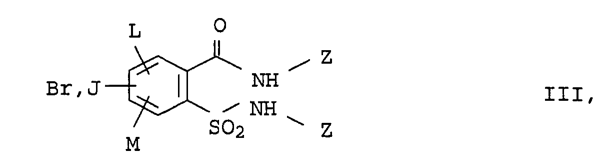 in Gegenwart eines Palladium-, Nickel-, Cobalt- oder Rhodium-Übergangsmetallkatalysators und einer Base mit Kohlenmonoxid und Wasser bzw. einem C₁-C₆-Alkohol unter erhöhtem Druck umsetzt.

Alkylreste in Formel I stehen insbesondere für niedermolekulare Alkylreste, z.B. mit 1 bis 6 Kohlenstoffatomen. Entsprechendes gilt für die Alkoxy- bzw. Alkylthioreste und Alkylsulfonylreste. Cycloalkyl ist z.B. C₃-C₈-Cycloalkyl wie Cyclopentyl, Cyclohexyl oder Cyclopropyl. Aryl steht z.B. für gegebenenfalls inerte Substituenten tragendes Phenyl. Aralkyl steht z.B. für gegebenenfalls inerte Substituenten tragendes Phenyl-C₁-C₄-alkyl wie Benzyl oder Phenethyl.

### Reaktionsgleichung:

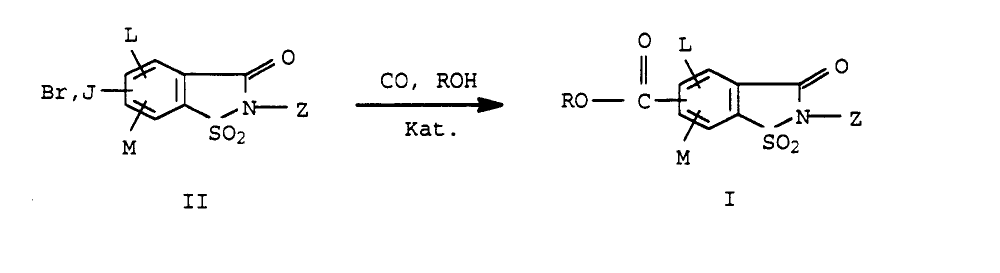

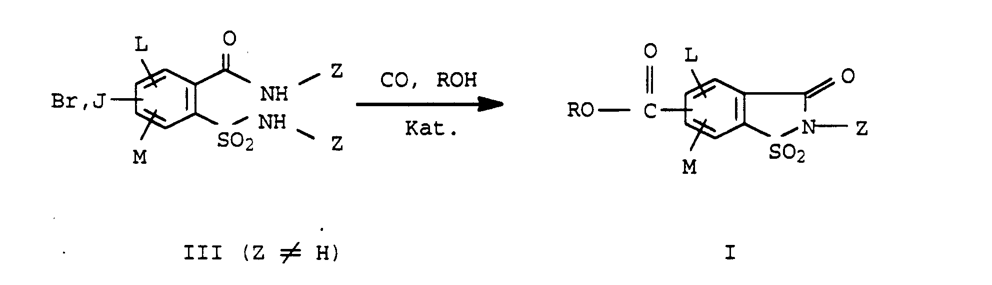

Die übergangsmetallkatalysierte Überführung von Halogenaromaten in die entsprechenden Carbonyl- bzw. Carboxylverbindungen ist mittels Carbonylierungsreagenzien an sich bekannt z. B. aus US 2,640,071; US 3,988,358; US 4,845,273; Urata et al. in J. Org. Chem. 56 (1991), 4320ff; Pri-Bar, Buchman in J. Org. Chem. 53 (1988), 624ff; US 4,990,657, GB-A 2,261,662 und M. Foa et al., J. Organometallic Chem. 285 (1985), 293ff.

Die Anwendbarkeit dieser Methode im Fall der speziellen Ausgangsstoffe II und III ist jedoch überraschend. Der Erfolg des erfindungsgemäßen Verfahrens war insbesondere aufgrund der vorhandenen funktionellen Gruppen in den Ausgangsstoffen nicht zu erwarten gewesen. Ferner war nicht zu erwarten gewesen, daß man über den erfindungsgemäßen Weg den Ausgangsstoff III unter Abspaltung des primären Amins ZNH₂ direkt in das carboxylierte Saccharinderivat I überführen könnte.

Die Katalysatoren Nickel, Cobalt, Rhodium und insbesondere Palladium können metallisch oder in Form üblicher Salze wie in Form von Halogenverbindungen, z.B. PdCl₂, RhCl₃ · 3H₂O, Acetaten, z.B. Pd(OAc)₂, Cyaniden usw. in den bekannten Wertigkeitsstufen vorliegen. Ferner können Metallkomplexe mit tertiären Phosphinen, Metallalkylcarbonyle, Metallcarbonyle, z.B. Co₂(CO)₈, Ni(CO)₄, Metallcarbonyl-Komplexe mit tert. Phosphinen, z.B. (PPh₃)₂Ni(CO)₂ oder mit tertiären Phosphinen komplexierte Übergangsmetallsalze vorliegen. Die letztgenannte Ausführungsform ist insbesondere im Fall von Palladium als Katalysator bevorzugt. Dabei ist die Art der Phosphinliganden breit variabel. Beispielsweise lassen sie sich durch folgende Formeln wiedergeben: 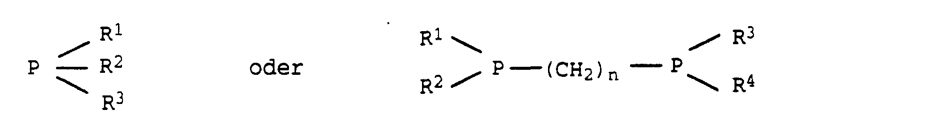 wobei n die Zahlen 1, 2, 3 oder 4 bedeutet und die Reste R¹ bis R⁴ für niedermolekulares Alkyl, z.B. C₁-C₆-Alkyl, Aryl, C₁-C₄-Alkylaryl, z.B. Benzyl, Phenethyl oder Aryloxy stehen. Aryl ist z.B. Naphthyl, Anthryl und vorzugsweise gegebenenfalls substituiertes Phenyl, wobei man hinsichtlich der Substituenten nur auf deren Inertheit gegenüber der Carboxylierungsreaktion zu achten hat, ansonsten können sie breit variiert werden und umfassen alle inerten C-organischen Reste wie C₁-C₆-Alkylreste, z.B. Methyl, Carboxylreste wie COOH, COOM (M ist z.B. ein Alkali-, Erdalkalimetall oder Ammoniumsalz), oder C-organische Reste über Sauerstoff gebunden wie C₁-C₆-Alkoxyreste.

Die Herstellung der Phosphinkomplexe kann in an sich bekannter Weise z.B. wie in den eingangs genannten Dokumenten, beschrieben hergestellt werden. Beispielsweise geht man von üblichen kommerziell erwerblichen Metallsalzen wie PdCl₂ oder Pd(OCOCH₃)₂ aus und fügt das Phosphin z.B. P(C₆H₅)₃, P(n-C₄H₉)₃, PCH₃(C₆H₅)₂, 1,2-Bis(diphenylphosphino)ethan hinzu. Beispielhaft seien folgende Katalysatoren genannt: 1,3-Bis(diisopropylphosphino)propan, Tri-p-anisylphosphin, Tri-o-tolylphosphin, 1,2-Bis(diphenylphosphino)butan, Triphenylphosphit.

Der Katalysator kann auch auf einem polymeren Träger gebunden sein. Die Herstellung solcher Katalysatoren ist u.a. im US-Patent 5,034,534 oder US 4,426,318 beschrieben.

Die Menge an Phosphin, bezogen auf das Übergangsmetall, beträgt üblicherweise 0 bis 20, insbesondere 0,1 bis 10 Molequivalente, besonders bevorzugt 1 bis 5 Molequivalente.

Die Menge an Übergangsmetall ist nicht kritisch. Natürlich wird man aus Kostengründen eher eine geringe Menge, z.B. von 0,1 bis 10 Mol.-%, insbesondere 1 bis 5 Mol.-%, bezogen auf den Ausgangsstoff II bzw. III verwenden.

Zur Herstellung der Saccharincarbonsäuren, d.h. R=H, führt man die Umsetzung mit Kohlenmonoxid und mindestens äquimolaren Mengen an Wasser, bezogen auf die Ausgangsstoffe II bzw. III durch. Zur Herstellung der Ester, d.h. R=OC₁-C₆-Alkyl, z.B. OCH₃, OC₂H₅, O-n-C₃H₇, O-n-C₃H₇, O-n-C₄-H₉, O-i-C₄H₉, O-tert.-C₄-H₉, O-n-C₅H₁₁, O-n-C₆H₁₃, verwendet man vorteilhaft mindestens äquimolare Mengen des entsprechenden Alkohols. Der Reaktionspartner Wasser bzw. C₁-C₆-Alkyl-OH kann gleichzeitig auch als Lösungsmittel dienen, d.h. die maximale Menge ist nicht kritisch.

Es kann aber auch je nach Art der Ausgangsstoffe und der verwendeten Katalysatoren von Vorteil sein, anstelle des Reaktionspartners ein anderes inertes Lösungsmittel oder die für die Carboxylierung verwendete Base als Lösungsmittel zu verwenden. In diesem Fall wird der Reaktionspartner Wasser bzw. Alkohol üblicherweise in Mengen von 1 bis 10, insbesondere 1 bis 5 Molequivalenten, bezogen auf II bzw. III eingesetzt.

Als inerte Lösungsmittel kommen für Carboxylierungsreaktionen übliche Lösungsmittel wie Kohlenwasserstoffe, z.B. Toluol, Xylol, Hexan, Pentan, Cyclohexan, Einer z.B. Methyl-tert.-butylether, Tetrahydrofuran, Dioxan, Dimethoxyethan, substituierte Amide wie Dimethylformamid, persubstituierte Harnstoffe, wie Tetra-C₁-C₄-alkylharnstoffe oder Nitrile wie Benzonitril oder Acetonitril in Betracht.

In einer bevorzugten Ausführungsform des Verfahrens verwendet man einen der Reaktionspartner, insbesondere die Base im Überschuß, so daß kein zusätzliches Lösungsmittel erforderlich ist.

Für das Verfahren geeignete Basen sind alle inerten Basen, die den bei der Umsetzung freiwerdenden Jodwasserstoff bzw. Bromwasserstoff zu binden vermögen. Beispielsweise sind hier tertiäre Amine wie Triethylamin, cyclische Amine wie N-Methyl-Piperidin oder N,N-Dimethyl-Piperazin, Pyridin, Amide wie N,N-Dimethylformamid, Alkali- oder Erdalkalimetallhydroxide, -carbonate oder -hydrogencarbonate sowie tetraalkylsubstituierte Harnstoffderivate wie Tetra-C₁-C₄-alkylharnstoff, z.B. Tetramethylharnstoff zu nennen.

Die Menge an Base ist nicht kritisch, üblicherweise werden 1 bis 10, insbesondere 1 bis 5 Mol verwendet. Bei gleichzeitiger Verwendung der Base als Lösungsmittel wird die Menge in der Regel so bemessen, daß die Reaktionspartner gelöst sind, wobei man aus Praktikabilitätsgründen unnötig hohe Überschüsse vermeidet, um Kosten zu sparen, kleine Reaktionsgefäße einsetzen zu können und den Reaktionspartnern maximalen Kontakt zu gewährleisten.

Während der Umsetzung wird der Kohlenmonoxiddruck so eingestellt, daß immer ein Überschuß an CO, bezogen auf II bzw. III vorliegt. Vorzugsweise liegt der Kohlenmonoxiddruck bei Raumtemperatur bei 1 bis 250 bar, insbesondere 5 bis 150 bar CO.

Die Carbonylierung wird in der Regel bei Temperaturen von 20 bis 250°C, insbesondere bei 30 bis 150°C kontinuierlich oder diskontinuierlich durchgeführt. Bei diskontinuierlichem Betrieb wird zweckmäßigerweise zur Aufrechterhaltung eines konstanten Druckes kontinuierlich Kohlenmonoxid auf das Umsetzungsgemisch aufgepreßt.

Aus dem anfallenden Reaktionsgemisch können die Produkte in üblicher Weise, z.B. durch Destillation isoliert werden.

Die für die Umsetzung benötigten Ausgangsstoffe II bzw. III sind bekannt oder können in an sich bekannter Weise hergestellt werden. Sie können entweder durch Permanganatoxidation von iodsubstituierten 2-Methylbenzolsulfonamiden oder durch Sandmeyer-Reaktion aus Aminosacchariden erhalten werden. Aminosaccharine werden nach bekannten Methoden durch Reduktion von Nitrosacchariden erhalten, die ihrerseits entweder bekannt sind (Kastle, Amer. Chem. Journel 11, 184 (1889) oder DRP 551423 (1930) oder in literaturbekannter Weise aus geeigneten Nitrobenzolderivaten (Liebigs Ann. 669, 85 (1963)) oder Benzolsulfonamiden synthetisiert werden.

Darüber hinaus können sie analog den Herstellvorschriften aus den Beispielen 1 bis 12 erhalten werden.

Die Saccharinderivate I dienen zur Herstellung von Pflanzenschutzmitteln, insbesondere von Herbiziden der Struktur E wie sie in der zeitgleichen deutschen Anmeldung DE-A 44 27 995 beschrieben werden. 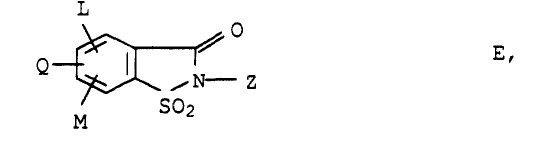 wobei die Substituenten folgende Bedeutung haben:
- L,M: Wasserstoff, C₁-C₄-Alkyl;
- Z: Wasserstoff, C₁-C₄-Alkyl;
- Q: ein Rest CO-J, wobei
- J: ein in Stellung 2 verknüpfter Cyclohexan-1,3-dionring der Formel A1 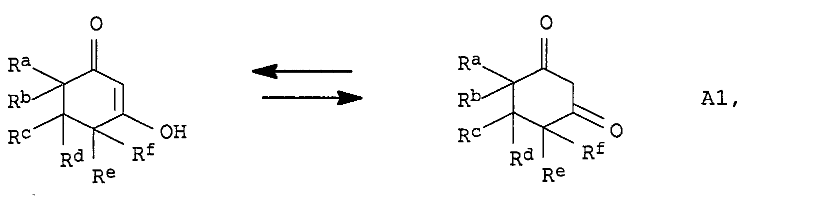 in welcher entweder R^{a} bis R^{f} Wasserstoff oder Methyl bedeuten, oder, wenn R^{a}, R^{b}, R^{c}, R^{e} und R^{f} Wasserstoff bedeuten, R^{d} 2-Ethylthiopropyl, Tetrahydropyranyl-3, Tetrahydropyranyl-4, Tetrahydrothiopyranyl-3 oder 1-Methylthio-cyclopropyl bedeutet,
oder, wenn R^{a}, R^{d}, R^{e} Wasserstoff und R^{f} Methyl bedeuten, R^{b} und R^{c} einen Dreiring bilden, so daß ein in Stellung 2 verknüpfter Bicyclo[4.1.0]heptanring der Formel A3 resultiert. 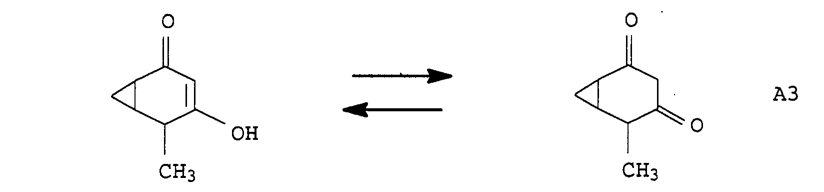
Zur Herstellung der Endprodukte E überführt man das Zwischenprodukt Ia in das entsprechende Säurechlorid A2 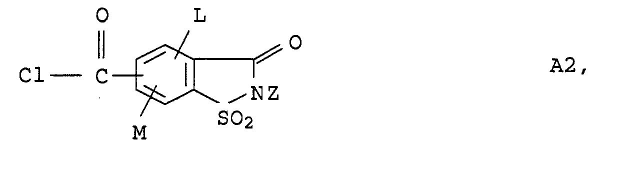 z.B. durch Umsetzung der Säure (R=H) mit Thionylchlorid. Anschließend wird der Ausgangsstoff A1 mit dem Zwischenprodukt A2 acyliert und der entstehende Enolester in Gegenwart eines Katalysators zum Endprodukt E umgelagert. Diese Reaktionssequenz läßt sich durch folgendes Reaktionsschema darstellen:

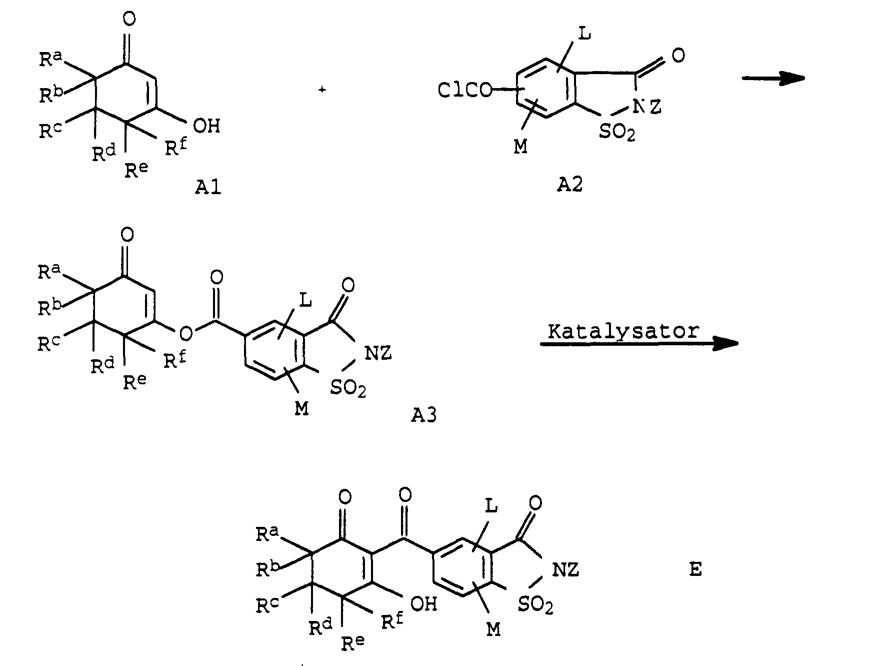

Der erste Schritt der Reaktionsabfolge, die Acylierung, erfolgt in allgemein bekannter Weise, z. B. durch Zugabe eines Säurechlorids der Formel A2 zur Lösung oder Suspension eines Cyclohexan-1,3-dions A2 oder A3 in Gegenwart einer Hilfsbase. Die Reaktanden und die Hilfsbase werden dabei zweckmäßig in äquimolaren Mengen eingesetzt. Als Hilfsbase eignen sich tertiäre Alkylamine, Pyridin oder Alkalicarbonate, während als Lösungsmittel Methylenchlorid, Diethylether, Toluol oder Essigsäureethylester verwendet werden können. Während der Zugabe des Säurechlorids wird die Reaktionsmischung auf 0 - 10°C gekühlt, danach wird bei einer Temperatur von 25 - 50°C gerührt, bis die Umsetzung beendet ist. Zur Aufarbeitung wird das Reaktionsgemisch in Wasser gegossen und mit Methylenchlorid extrahiert. Nach Trocknen der organischen Phase und Entfernung des Lösungsmittels wird der rohe Enolester ohne weitere Reinigung zur Umlagerung eingesetzt. Herstellungsbeispiele für Benzoyl-enolester von Cyclohexan-1,3-dionen findet man z. B. in EP-A 186 118 oder US 4,780,127.

Die Umlagerung der Enolester zu den Verbindungen der Formel E erfolgt vorteilhaft bei Temperaturen von 20°C bis 40°C in einem Lösungsmittel und in Gegenwart einer Hilfsbase sowie mit Hilfe einer Cyanoverbindung als Katalysator, wobei als Lösungsmittel Acetonitril, Methylenchlorid, 1,2-Dichlorethan, Essigsäureethylester oder Toluol verwendet werden. Bevorzugtes Lösungsmittel is Acetonitril. Als Hilfsbase eignen sich tertiäre Alkylamine, Pyridin oder Alkalicarbonate, die in äquimolarer Menge oder bis zu vierfachem Überschuß eingesetzt werden. Bevorzugte Hilfsbase ist Triethylamin in doppelter Menge. Als Katalysator eignen sich Kaliumcyanid oder Acetoncyanhydrin in einer Menge von 1 bis 50 Molprozent, bezogen auf den Enolester. Bevorzugt setzt man 10 Molprozent Acetoncyanhydrin zu. Beispiele zur cyanidkatalysiertei Umlagerung von Enolestern der Cyclohexan-1,3-dione findet man z.B. in EP-A 186 118 oder US 4,780,127.

Zur Aufarbeitung wird das Reaktionsgemisch z.B. mit verdünnten Mineralsäuren wie 5 proz. Salzsäure oder Schwefelsäure angesäuert und mit Methylenchlorid oder Essigsäureethylester extrahiert. Zur Reinigung wird der Extrakt mit kalter 5 - 10 proz. Alkalicarbonatlösung extrahiert, wobei das Endprodukt in die wäßrige Phase übergeht. Durch Ansäuern der wäßrigen Lösung wird das Produkt dei Formel E ausgefällt, oder erneut mit Methylenchlorid extrahiert, getrocknet und anschließend vom Lösungsmittel befreit.

Die als Ausgangsmaterial verwendeten 1,3-Diketone der Formeln A2 bzw. A3 sind bekannt oder können nach an sich bekannten Verfahren hergestellt werden. (vgl. EP-A 71 707, EP-A 142 741, EP-A 243 313, US 4 249 937 und WO 92/13821. Cyclohexandion-1,3, und Dimedon sind käufliche Verbindungen.

In analoger Weise können auch Saccharinderivate E hergestellt werden, bei denen der Cyclohexan-1,3-dionring durch einen Pyrazol-4-ylrest ersetzt ist. In diesem Fall geht man von den Ausgangsstoffen A4 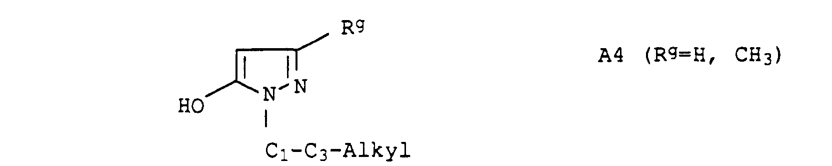 aus.

In der zeitgleichen deutschen Anmeldung DE-A 44 27 997 werden solche herbizid wirksamen Folgeprodukte beschrieben.

Die Saccharincarbonsäuren bzw. -ester der Formel I dienen nicht nur als Zwischenprodukte für die Herstellung herbizid wirksamer Folgeprodukte, sondern weisen selber eine gute herbizide Wirksar keit auf. Demgemäß ist die Verwendung der Verbindungen Ia als Herbizide bzw. ein Verfahren zur Bekämpfung unerwünschten Pflanzenwuchses mit den Verbindungen Ia 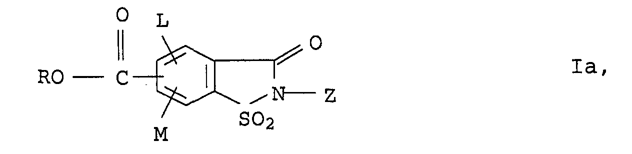 in der die Substituenten folgende Bedeutung haben:
- L, M: Wasserstoff, C₁-C₄-Alkyl,
- Z: Wasserstoff, C₁-C₄-Alkyl,
- R: H, C₁-C₆-Alkyl
ein weiteres Erfindungsmerkmal.

Darüber hinaus sind Gegenstand der Erfindung neue herbizid wirksame Saccharincarbonsäuren bzw. Carbonsäureester der Formel I 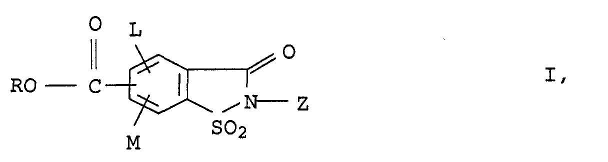 in der die Substituenten folgende Bedeutung haben:
- L, M: Wasserstoff, C₁-C₄-Alkyl,
- Z: Wasserstoff, C₁-C₄-Alkyl,
- R: H, C₁-C₆-Alkyl
mit der Maßgabe, daß Z nicht Methyl, Wasserstoff oder ein Alkali metall- oder Silberkation bedeuten, wenn L und M für Wasserstoff stehen, sowie ferner ausgenommen 5-Carboxy-7-methylsaccharin.

Besonders bevorzugt sind Carbonsäuren der Formel I (R=H) mit einer oder zwei weiteren Substituenten in Phenylring. Beispielsweise mit L=C₁-C₄-Alkyl, z.B. Methyl, und M = Wasserstoff oder C₁-C₄-Alkyl, z.B. Methyl.

Die Verbindungen I bzw. Ia können in Form ihrer landwirtschaftlich brauchbaren Salze vorliegen, wobei es auf die Art des Salze im allgemeinen nicht ankommt. Üblicherweise werden die Salze von solchen Basen in Betracht kommen, welche die herbizide Wirkung von I bzw. Ia' nicht negativ beeinträchtigen.

Als Salze eignen sich besonders diejenigen der Alkalimetalle, vorzugsweise die Natrium- und Kaliumsalze, die der Erdalkalimetalle, vorzugsweise Calcium-, Magnesium-, und die der Übergangsmetalle, vorzugsweise Silber-, Kupfer-, Zink- und Eisensalze sowie die Ammoniumsalze, die ein bis drei C₁-C₄-Alkyl, Hydroxy-C₁-C₄-alkylsubstituenten und/oder einen Phenyl- oder Benzylsubstituenten tragen können, vorzugsweise Diisopropylammonium-, Tetramethylammonium-, Tetrabutylammonium-, Trimethylbenzylammonium-, und Trimethyl-(2-hydroxyethyl)-ammoniumsalze, die Phosphoniumsalze, die Sulfoniumsalze, vorzugsweise Tri-(C₁-C₄-)alkylsulfoniumsalze, und die Sulfoxoniumsalze, vorzugsweise Tri- (C₁-C₄-)alkylsulfoxoniumsalze.

Die Verbindungen I oder Ia bzw. die sie enthaltenden herbiziden Mittel sowie deren umweltverträgliche Salze von beispielsweise Alkalimetallen, Erdalkalimetallen oder Ammoniak und Aminen bzw. die sie so enthaltenden herbiziden Mittel können in Kulturen wie Weizen, Reis, Mais, Soja und Baumwolle Unkräuter und Schadgräser sehr gut bekämpfen, ohne die Kulturpflanzen nennenswert zu schädigen. Dieser Effekt tritt vor allem bei niedrigen Aufwandmengen auf.

Unter Berücksichtigung der Vielseitigkeit der Applikationsmethoden können die Verbindungen I, Ia bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum,
Prunus avium, Prunus persica, Pyrus communis, Ribes sylvestre, Ricinus communis, Saccharum officinarum, Secale cereale, Solanun tuberosum, Sorghum bicolor (s. vulgare), Theobroma cacao, Trifolium pratense, Triticum aestivum, Triticum durum, Vicia faba, Vitis vinifera, Zea mays.

Darüber hinaus lassen sich die Verbindungen I und Ia auch in Kulturen, die durch Züchtung und/oder mittels gentechnischer Methoden gegen die Wirkung von I bzw. Ia oder anderen Herbiziden weitgehend resistent gemacht wurden, einsetzen.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeitnicht getroffen werden, während die Wirkstoffe auf die Blätter darunter wachsender unerwünschter Pflanzen oder die unbedeckte Bodenfläche gelangen (post-directed, lay-by).

Die Verbindungen I, Ia bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline oder deren Derivate, alkylierte Benzole und deren Derivate, Alkohole wie Methanol, Ethanol, Propanol, Butanol und Cyclohexanol, Ketone wie Cyclohexanon oder stark polare Lösungsmittel, z.B. Amine wie N-Methylpyrrolidon, oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen von 0,01 bis 95 Gew.-%, vorzugsweise 0,5 bis 90 Gew.-%, Wirkstoff. Die Wirkstoffe werden dabei in einer Reinheit von 90 % bis 100 %, vorzugsweise 95 % bis 100 % (nach NMR-Spektrum) eingesetzt. Die erfindungsgemäßen Verbindungen I können beispielsweise wie folgt formuliert werden:
- I.: 20 Gewichtsteile der Verbindung Nr. 1.002 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
- II.: 20 Gewichtsteile der Verbindung Nr. 1.002 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- III.: 20 Gewichtsteile des Wirkstoffs Nr. 1.002 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- IV.: 20 Gewichtsteile des Wirkstoffs Nr. 1.002 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
- V.: 3 Gewichtsteile des Wirkstoffs Nr. 1.002 werden mit 97 Gewichtsteilen feinteiligem Kaolin vermischt. Man erhält auf diese Weise ein Stäubemittel, das 3 Gew.-% des Wirkstoffs enthält.
- VI.: 20 Gewichtsteile des Wirkstoffs Nr. 1.002 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Zur Verbreiterung des Wirkungsspektrums und zur Erzielung synergistischer Effekte können die Saccharinderivate Ia bzw. Ia' mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen Ia oder Ia' allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

### Herstellungsbeispiele

### A) Herstellung der Ausgangsstoffe

### 1. 2-Methyl-6-acetaminobenzoesäure

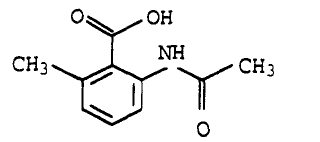

Zu einer Lösung von 24,8 g (0,62 Mol) NaOH in 500 ml Wasser gibt man 90,6 g (0,6 Mol) 6-Methylanthranilsäure und tropft dann 63,4 g (0,62 Mol) Acetanhydrid zu. Nach einer Stunde Nachrühren wird unter Kühlung mit konz. HCl auf pH 3 angesäuert, der ausfallende Niederschlag wird abgesaugt, mit Wasser gewaschen und bei 50°C i.Vak. getrocknet. Ausbeute: 107 g (0,55 Mol) = 92 % d.Th., Fp.: 189 - 190°C

### 2. 2-Methyl-3-nitro-6-acetaminobenzoesäure

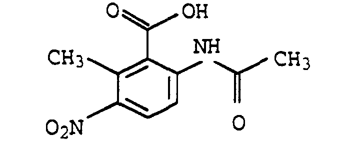

Man legt 271 ml 98 proz. Salpetersäure bei - 5°C vor und trägt portionsweise 106 g (0,55 Mol) der unter 1. hergestellten 2-Methyl-6-acetaminobenzoesäure ein. Nach einer Stunde Nachrühren bei 10°C wird das Reaktionsgemisch in eine Mischung aus 540 g Eis und 270 ml Wasser gegossen. Der ausgefallene Niederschlag wird abgesaugt, mit Wasser gewaschen und bei 50°C i.Vak. getrocknet. Ausbeute: 75,6 g (0,317 Mol) = 58 % d.Th., Fp.: 190 - 191°C

Aus dem Filtrat scheidet sich nach längerem Stehen das in 3-Stellung nitrierte Isomere ab:
Ausbeute: 21,3 g (0.089 Mol) = 16 % d.Th., Fp.: 180 - 182°C

### 3. 2-Methyl-3-nitro-6-aminobenzoesäure

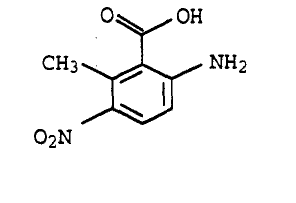

Man legt 450 ml 2-N NaOH vor und fügt 75,6 g (0,317 Mol) 2-Methyl-3-nitro-6-acetaminobenzoesäure zu. Anschließend erwärmt man die Reaktionsmischung auf 95°C und läßt eine Stunde bei dieser Temperatur rühren. Nach Abkühlung auf 10°C wird durch Zugabe von 425 ml 2-N HCl angesäuert, saugt den ausfallenden Niederschlag ab, wäscht mit Wasser und trocknet bei 50°C i.Vak..
Ausbeute: 50,7 g (0,258 Mol) = 82 % d.Th., Fp.: 183 - 184°C

### 4. 2-Methyl-3-nitro-6-aminobenzoesäuremethylester

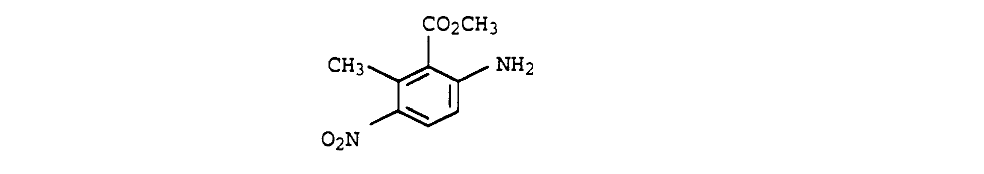

Man löst 49,7 g (0,253 Mol) 2-Methyl-3-nitro-6-aminobenzoesäure in 380 ml Aceton und fügt 43 g (0,51 Mol) Natriumhydrogencarbonat zu. Anschließend wird zum Sieden erhitzt, bis die CO₂-Entwicklung abgeschlossen ist. Zu der so erhaltenen Suspension des Natriumsalzes von 2-Methyl-3-nitro-6-aminobenzoesäure tropft man anschließend bei Siedetemperatur des Acetons 35,3 g (0,28 Mol) Dimethylsulfat im Laufe von zwei Stunden zu, refluxiert anschließend noch drei Stunden und läßt dann abkühlen. Nach Eingießen der Reaktionsmischung in 1,8 1 Wasser wird mit Methylenchlorid extrahiert. Nach Trocknen der organischen Phase wird eingeengt. Der erhaltene Feststoff ist für die Folgeumsetzung genügend rein (NMR).
Ausbeute: 50 g (0,238 Mol) = 94 % d.Th., Fp.: 92 - 94°C

### 5. 2-Methoxycarbonyl-3-methyl-4-nitro-benzolsulfonsäurechlorid

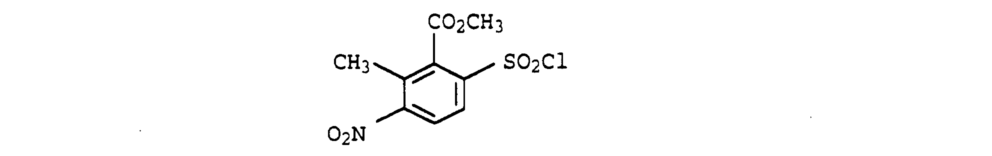

Man löst unter Erwärmen 58,5 g (0,278 Mol) 2-Methyl-3-nitro-6-amino-benzoesäure-methylester in 280 ml Eisessig und gießt diese Lösung bei 15 - 20°C in 85 ml konz. HCl. Dann tropft man bei 5 - 10°C eine Lösung von 19,3 g (0,28 Mol) Natriumnitrit in 60 ml Wasser zu und läßt 30 min bei 5°C nachrühren. Anschließend tropft man diese Diazoniumsalzlösung in eine Lösung von 374 g SO₂ in 750 ml Eisessig, die 14 g CuCl₂ (in 30 ml Wasser gelöst) enthält. Nach Beendigung der Stickstoffentwicklung wird noch 15 min nachgerührt und dann in 1,4 1 Eiswasser gegossen. Das Sulfonsäurechlorid wird durch Extraktion mit 1,2 1 Methylenchlorid abgetrennt. Nach Trocknen und Einengen der organischen Phase erhält man 73 g (0,25 Mol) (= 90 % d.Th.) eines Öls, das nach NMR (in CDCl₃) reines 2-Methoxycarbonyl-3-methyl-4-nitro-benzolsulfonsäurechlorid ist.

### 6. 4-Methyl-5-nitrosaccharin

### 4-Methyl-5-nitro-1,1,3-trioxo-2,3-dihydro-1λ⁶benz[d]isothiazol (Beilstein-Nomenklatur)

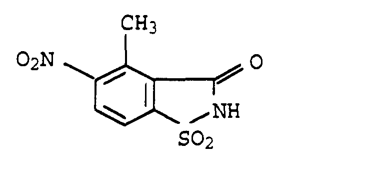

Man legt 104 ml 25 proz. Ammoniaklösung vor, fügt 100 ml Wasser zu und tropft dann bei 10°C eine Lösung von 48,7 g (0,166 Mol) 2-Methoxycarbonyl-3-methyl-4-nitro-benzolsulfonsäurechlorid in 70 ml Tetrahydrofuran zu. Nach drei Stunden Rühren bei 25°C wird am Rotationsverdampfer eingeengt, um Wasser und THF zu entfernen. Der verbleibende Rückstand wird mit Essigester verrührt, abgesaugt und mit Essigester gewaschen. Nach dem Trocknen i.Vak. erhält man 34 g (0,131 Mol) = 79 % d.Th. eines weißen Feststoffs mit Fp.: 312°C (Zers.)

### 7. 2,4-Dimethyl-5-nitrosaccharin

Diese Substanz kann durch nachträgliche Methylierung des unter 6. erhaltenen Saccharins mit Dimethylsulfat in Gegenwart von NaOH hergestellt werden.

### 8. 3-Methyl-4-nitro-2-(N'-methyl)carboxamido-N-methylbenzolsulfonamid

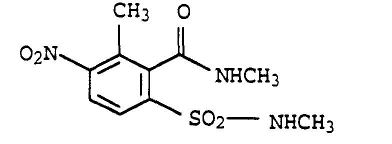

Man gießt 50 ml Wasser in 50 ml 40 proz. Methylamin-Lösung und tropft bei 10°C eine Lösung von 24,3 g (83 mMol) 2-Methoxycarbonyl-3-methyl-4-nitro-benzolsulfonsäurechlorid in 35 ml THF zu. Nach einer Stunde Rühren bei 25°C werden alle flüchtigen Bestandteile am Rotationsverdampfer abgezogen, der Rückstand wird mit Essigester extrahiert, die organische Phase mit Wasser gewaschen, getrocknet und eingeengt. Der verbleibende Rückstand kristallisiert nach längerem Stehen.
Ausbeute: 10,3 g (40 mMol = 48 % d.Th.), Fp.: 125 - 126°C, nach Umkristallisation aus Essigester Fp.: 144 - 145°C.

### 9. 4-Methyl-5-amino-1,1,3-trioxo-2,3-dihydro-1λ⁶benz[d]isothiazol (Beilstein-Nomenklatur)

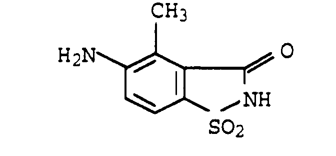

Man löst unter Erwärmen auf 45°C 33,6 g (0,13 Mol) 4-Methyl-5-nitro-1,1,3-trioxo-2,3-dihydro-1λ⁶benz[d]isothiazol in 1,2 l Wasser und fügt 5 g Pd/C (10 proz. auf Aktivkohle) zu. Anschließend leitet man unter heftigem Rühren Wasserstoffgas ein (drucklose Hydrierung). Im Laufe von 4,5 Stunden werden 9 l H₂ aufgenommen. Nach Abkühlen auf 25°C wird vom Katalysator abfiltriert, am Rotavapor auf 200 ml Volumen eingeengt und dann auf pH 1 angesäuert. Der ausgefallene Niederschlag wird abgesaugt, mit Wasser gewaschen und bei 50°C i.Vak. getrocknet. Man erhält 23,4 g (0,11 mol = 85 % d.Th.) eines weißen Feststoffs mit Fp.: 272 - 273°C

### 10. 4-Methyl-5-iod-1,1,3-trioxo-2,3-dihydro-1λ⁶benz[d]isothiazol (Beilstein-Nomenklatur)

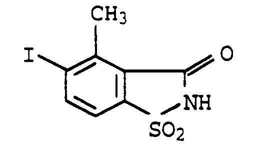

Man legt eine Mischung von 205 ml Eisessig, 160 ml Wasser und 40 ml konz. HCl vor und trägt unter Rühren 23,4 g (0,11 Mol) 4-Methyl-5-amino-1,1,3-trioxo-2,3-dihydro-1λ⁶benz[d]isothiazol bei 15 - 20°C ein. Zu der entstandenen Suspension tropft man bei 5 - 10°C 7,9 g (0,115 Mol) Natriumnitrit und läßt 30 min bei 5°C nachrühren. Das als Suspension vorliegende Diazoniumsalz wird dann portionsweise in eine auf 50°C erwärmte Lösung von 19,1 g (0,115 Mol) Kaliumiodid in 170 ml Wasser getropft, wobei Stickstoff entsteht. Nach Abkühlen auf Raumtemperatur wird das ausgefallene Produkt durch Absaugen isoliert, mit Wasser gewaschen und bei 50°C i.Vak. getrocknet. Man erhält 32,5 g (0,1 Mol = 91 % d.Th.) eines Feststoffs mit Fp.: 257 - 258°C. Eine Verbrennungsanalyse ergab einen Iodgehalt von 38,5 % (Theorie 39,3 %).

Das Produkt ist für die Folgeumsetzungen genügend rein.

### 11. 4-Amino-3-methyl-2-(N'-methyl)carboxamido-N-methylbenzolsulfonamid

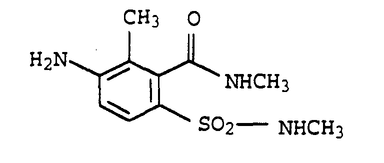

Analog dem bei Beispiel 9 beschriebenen Vorgehen wurde das nach Beispiel 8 erhaltene 3-Methyl-4-nitro-2-(N'-methyl)carboxamido-N-methyl-benzolsulfonamid drucklos hydriert. Man erhielt in 93 % Ausbeute das Anilinderivat o.g. Struktur mit Fp.: 217 - 218°C

### 12. 3-Methyl-4-iod-2-(N'-methyl)carboxamido-N-methylbenzolsulfonamid

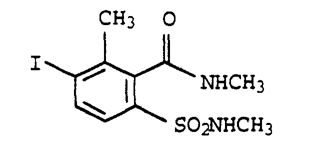

Nach dem unter Beispiel 10 beschriebenen Vorgehen wurde die voranstehende Verbindung diazotiert und durch Umsetzung mit KI zu dem Iodbenzolderivat nebenstehender Struktur umgesetzt. Ausbeute: 95 % d.Th., Fp.: 60 - 62°C

### B) Herstellung der Endprodukte I

### 13. 4-Methyl-1,1,3-trioxo-2,3-dihydro-1λ⁶benz[d]isothiazol-5-carbonsäure

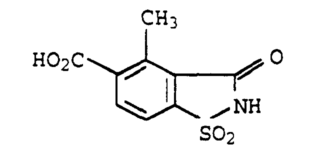

6,4 g (0,02 mol) 4-Methyl-5-iod-1,1,3-trioxo-2,3-dihydro-1-λ⁶benz[d]isothiazol werden in 70 ml Tetramethylharnstoff und 30 ml Wasser gelöst und mit 0,7 g Bis(triphenylphosphin)palladiumdichlorid versetzt. Das Gemisch wird in einem 300-ml-Autoklaven auf 100°C erhitzt und 36 h bei einem Druck von 100 bar Kohlenmonoxid gerührt.

Zur Aufarbeitung filtriert man und entfernt Wasser und Tetramethylharnstoff destillativ im Hochvakuum. Der Rückstand wird in Methyl-tert.-butylether (MTBE) aufgenommen, mit NaHCO₃-Lsg. extrahiert und nach dem Ansäuern mit HCl wieder mit MTBE extrahiert. Nach dem Einengen erhält man 2,8 g 4-Methyl-1,1,3-trioxo-2,3-dihydro-1-λ⁶benz[d]isothiazol-5-carbonsäure (58 % d. Th.).
¹H-NMR (DMSO, 400,1 MHz): 2,85 (3H, s); 8,05 (1H, d); 8,2 (1H, d);
¹³C-NMR (DMSO, 100,6 MHz): 167,4 (CO); 161,3 (CO); 141,6 (quart. C); 139,7 (quart. C); 138,7 (quart. C); 135,6 (CH); 125,4 (quart. C); 118,5 (CH); 15,4 (CH₃).

### 14. 4-N-Dimethyl-1,1,3-trioxo-2,3-dihydro-1-λ⁶benz[d]isothiazol-5-carbonsäure

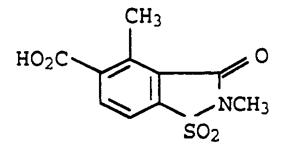

7,3 g (0,02 mol) 3-Methyl-4-iod-2-(N'-methyl)carboxamido-N-methylbenzol-sulfonamid werden zusammen mit 0,69 g Bis(tri-phenylphosphin)palladiumdichlorid, 30 ml Wasser und 70 ml Tetramethylharnstoff in einem 300-ml-Autoklaven vorgelegt.
Das Gemisch wird auf 100 °C erhitzt und 36 h bei einem Druck von 100 bar Kohlenmonoxid gerührt.

Nach Aufarbeitung wie in Beispiel 13 beschrieben erhält man 4,1 g der Titelverbindung (0,014 mol = 72 % d. Th.).
¹H-NMR (DMSO, 400,1 MHz): 2,9 (3H, s); 3,15 (3H, s); 8,2 (2H, 2d); 14,0 (1H, s)
¹³C-NMR (DMSO, 100,6 MHz): 167,3 (CO); 158,6 (CO); 139,7 (quart. C); 139,1 (quart. C); 138,9 (quart. C); 135,5 (CH); 124,6 (quart. C); 119,0 (CH); 22,9 (CH₃); 15,6 (CH₃).

In analoger Weise können die in Tabelle 1 zusammengestellten Saccharincarbonsäuren erhalten werden. Die in Tabelle 1 für einen Substituenten genannten Gruppen stellen außerdem für sich betrachtet - unabhängig von der speziellen Kombination mit anderen Substituenten, in der sie genannt sind - eine besonders bevorzugte Definition des betreffenden Substituenten dar.

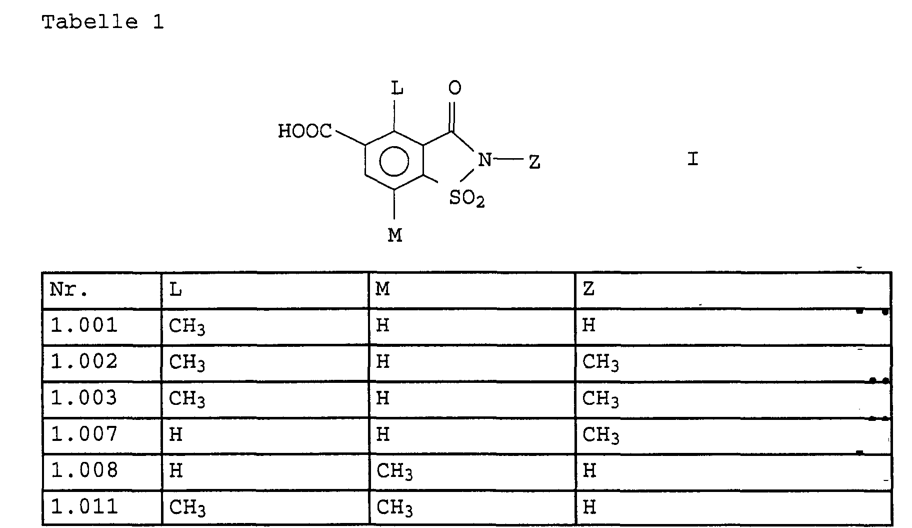

### C) Umsetzung der Verbindungen I zu herbizid wirksamen Folgeprodukten.

### 15. 2,4-Dimethyl-saccharin-5-carbonsäurechlorid

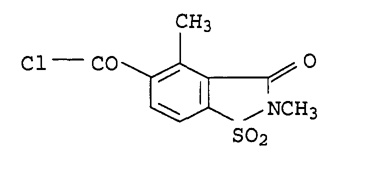

Man suspendiert 3,8 g (14,9 mMol) der 4,N-Dimethyl-1,1,3-trioxo-2,3-dihydro-1λ⁶benz[d]isothiazol-5-carbonsäure in 100 ml Toluol, erwärmt auf 80°C und tropft 3,5 g (29,8 mMol) Thionylchlorid zu. Nach zwei Stunden Refluxieren wird heiß dekantiert und das Reaktionsgemisch am Rotationsverdampfer eingeengt.
Ausbeute: 74 % d.Th., Fp.: 149 - 150°C.

### 16. Acylierung von Cyclohexandion

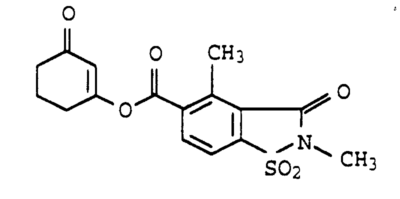

Zu einer Suspension von 1,23 g (10,9 mMol) Cyclohexandion-1,3 in 50 ml Methylenchlorid gießt man 1,21 g (12 mMol) Triethylamin und tropft anschließend bei 25°C eine Lösung von 3 g (10,9 mMol) 4,N-Dimethyl-1,1,3-trioxo-2,3-dihydro-1λ⁶benz[d]isothiazol-5-carbonsäure-chlorid in 60 ml Methylenchlorid zu. Anschließend wird 7 Stunden bei 40°C gerührt. Nach dem Abkühlen werden 60 ml Wasser zugegossen, in einem Scheidetrichter die Methylenchloridphase abgetrennt und über Magnesiumsulfat getrocknet. Der nach dem Abziehen des Lösungsmittels verbleibende amorphe Rückstand (2,5 g) ist der Enolester nebenstehender Struktur, der ohne weitere Reinigung in der nächsten Stufe umgelagert wird.

### 17. Umlagerung zum Endprodukt E

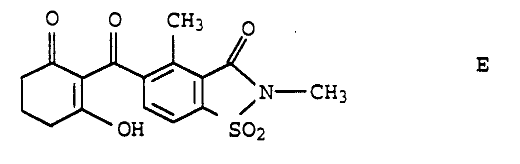

2,5 g (7,2 mMol) des voranstehenden Enolesters werden in 80 ml Acetonitril gelöst, mit 3,5 ml Triethylamin und dann mit 0,33 g (4 mMol) Acetoncyanhydrin versetzt und 16 h gerührt. Danach werden 24,5 g 5 proz. HCl zugegeben und das Reaktionsgemisch mit 100 ml Methylenchlorid extrahiert. Anschließend wird die organische Phase mit 5 proz. Kaliumcarbonatlösung extrahiert, wobei das Produkt in die wäßrige Phase übergeht. Durch Ansäuern der alkalisch-wäßrigen Lösung mit konz. HCl wird ein gummiartiger Feststoff ausgefällt, der nach Anreiben mit Diisopropylether auskristallisiert. Nach Waschen mit Petrolether wird i.Vak. getrocknet.
Ausbeute: 0,88 g (35 % d.Th.)

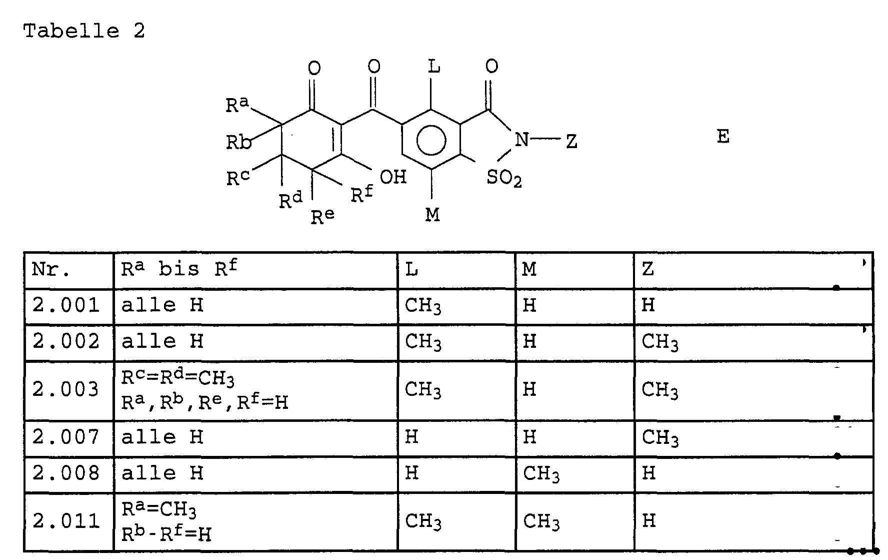

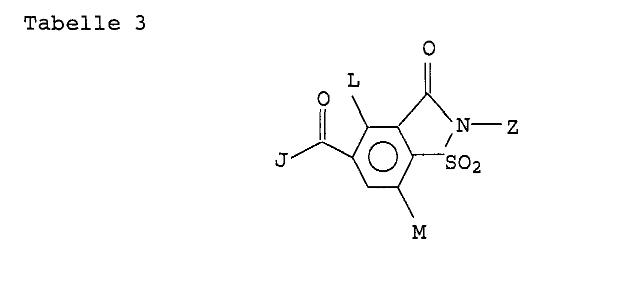

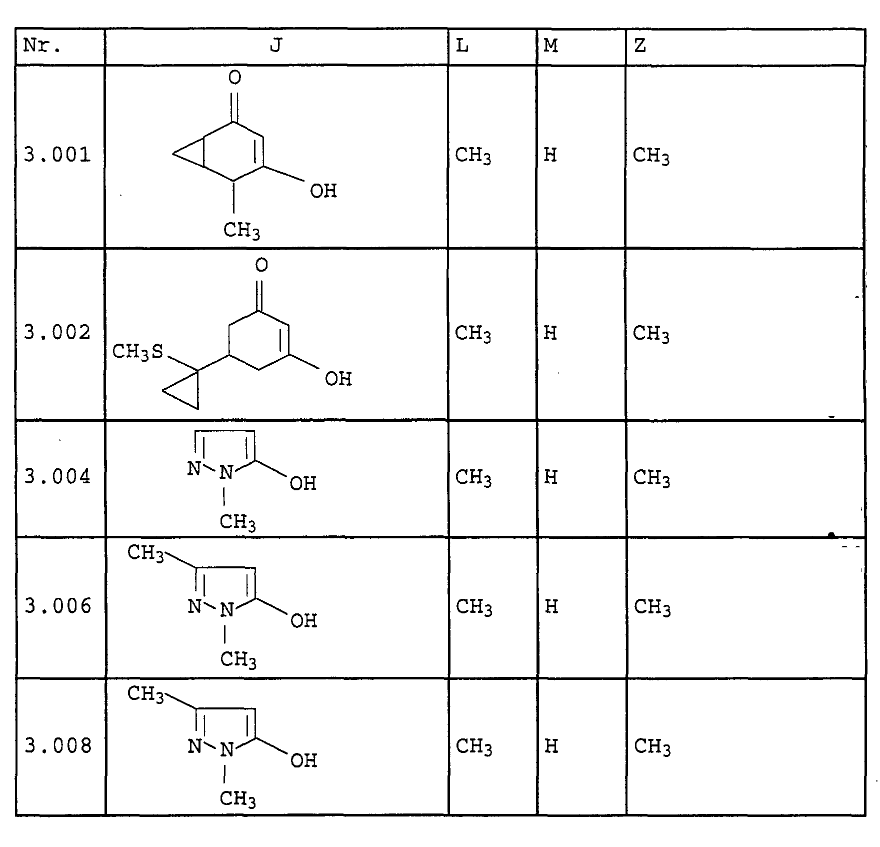

Anwendungsbeispiele zur herbiziden Wirksamkeit der Verbindungen I bzw. Ia

Die herbizide Wirkung der Saccharincarbonsäuren bzw. -ester der Formel I bzw. Ia ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung betrug 3,0 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | | |
|---|---|---|
| Lateinischer Name | Deutscher Name | Englischer Name |
| Echinocloa crus-galli | Hühnerhirse | barnyardgrass |
| Setaria italica | Kolbenhirse | foxtail millet |

Bei einer Aufwandmenge von 3,0 kg/ha a.S. lassen sich mit der Verbindung aus Beispiel 1.002 unerwünschte Pflanzen im Nachauflaufverfahren sehr gut bekämpfen.

## Patentansprüche

1. Verfahren zur Herstellung von Saccharincarbonsäuren und -carbonsäureestern der Formel I, 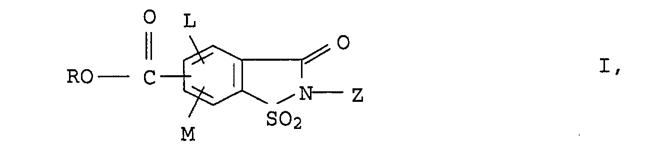 in der die Substituenten folgende Bedeutung haben:
L, M Wasserstoff, C₁-C₄-Alkyl;
Z Wasserstoff, C₁-C₄-Alkyl;
R H, C₁-C₆-Alkyl,
**dadurch gekennzeichnet, daß** man entsprechende brom- oder iodsubstituierte Saccharinderivate der Formel II 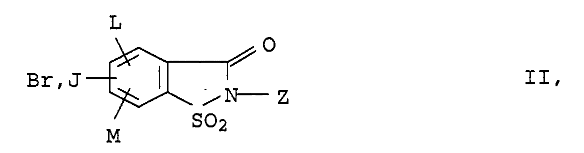 in der L, M und Z die obengenannte Bedeutung haben, oder im Falle von Z ≠ H Verbindungen der Formel III 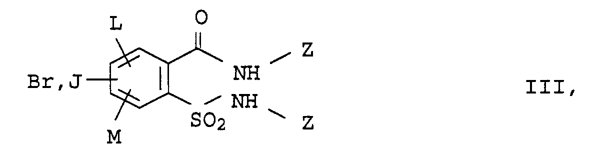 in Gegenwart eines Palladium-, Nickel-, Cobalt- oder Rhodium-Übergangsmetallkatalysators und einer Base mit Kohlenmonoxid und Wasser bzw. einem C₁-C₆-Alkohol unter erhöhtem Druck umsetzt.

2. Verfahren nach Anspruch 1, **dadurch gekennzeichnet, daß** man für Carbonylierungsreaktionen übliche Palladiumkatalysatoren verwendet.

3. Verfahren nach Anspruch 1, **dadurch gekennzeichnet, daß** man die Carboxylierung in Gegenwart von Palladiumkatalysatoren, die Phosphinliganden aus tertiären Phosphinen enthalten, vornimmt.

4. Verfahren nach Anspruch 1, **dadurch gekennzeichnet, daß** man als Base tertiäre Amine oder Tetraalkyl substituierte Harnstoffderivate verwendet.

5. Verfahren nach Anspruch 1, **dadurch gekennzeichnet, daß** man als Base Tetramethylharnstoff verwendet.

6. Verfahren nach den Ansprüchen 1 und 5, **dadurch gekennzeichnet, daß** man die Base gleichzeitig als Lösungsmittel verwendet.

7. Verfahren nach Anspruch 1, **dadurch gekennzeichnet, daß** man den Reaktionspartner Wasser oder Alkohol gleichzeitig als Lösungsmittel verwendet.

8. Verfahren nach Anspruch 1, **dadurch gekennzeichnet, daß** man die Carboxylierung bei einer Temperatur von 20 bis 200°C vornimmt.

9. Saccharincarbonsäuren bzw. -carbonsäureester der Formel I 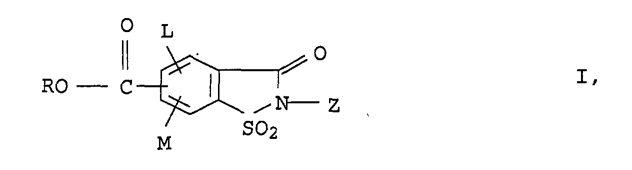 in der die Substituenten folgende Bedeutung haben:
L, M Wasserstoff, C₁-C₄-Alkyl,
Z Wasserstoff, C₁-C₄-Alkyl,
R H, C₁-C₆-Alkyl,
mit der Maßgabe, daß Z nicht Methyl oder Wasserstoff oder ein Alkalimetall- oder Silberkation bedeutet, wenn L und M für Wasserstoff stehen, sowie ferner ausgenommen 5-Carboxy-7-methylsaccharin.

10. Herbizide Mittel, enthaltend die Verbindungen Ia gemäß Anspruch 9 und übliche inerte Trägerstoffe.

11. Verfahren zur Bekämpfung unerwünschten Pflanzenwuchses,
**dadurch gekennzeichnet, daß** man eine herbizid wirksame Menge einer Saccharincarbonsäure- bzw. eines -carbonsäureesters der Formel Ia 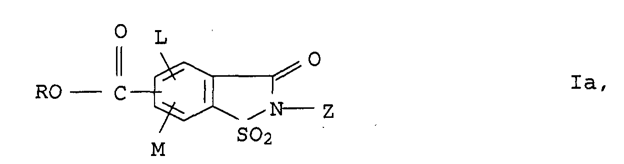 in der die Substituenten folgende Bedeutung haben:
L, M Wasserstoff, C₁-C₄-Alkyl,
Z Wasserstoff, C₁-C₄-Alkyl,
R H, C₁-C₆-Alkyl
oder ein umweltverträgliches Salz der Verbindungen Ia auf die Pflanzen oder deren Lebensraum einwirken läßt.

## Claims

1. A process for preparing saccharincarboxylic acids and ―carboxylic acid esters of the formula I 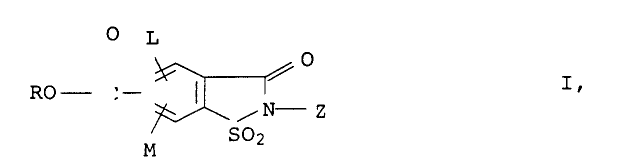 where the substituent have the following meanings:
L and M are hydrogen or C₁-C₄-alkyl,
Z is hydrogen or C₁-C₄-alkyl,
R is H or C₁-C₆-alkyl,
which comprises reacting corresponding bromo- or iodo-substituted saccharin derivatives of the formula II 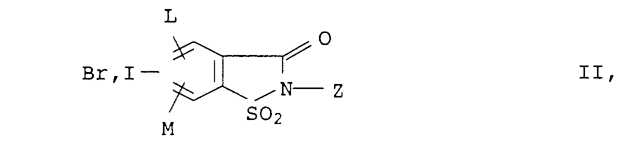 where L, M and Z have the abovementioned meanings, or if Z ≠ H, compounds of the formula III 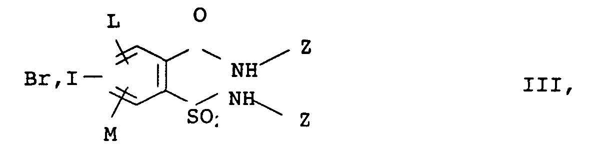 in the presence of a palladium, nickel, cobalt or rhodium transition metal catalyst and of a base with carbon monoxide and water or a C₁-C₆-alcohol under elevated pressure.

2. A process as claimed in claim 1, wherein palladium catalysts customary for carbonylation reactions are used.

3. A process as claimed in claim 1, wherein the carboxylation is performed in the presence of palladium catalysts which contain phosphine ligands from tertiary phosphines.

4. A process as claimed in claim 1, wherein the base used is a tertiary amine or tetraalkyl-substituted urea derivative.

5. A process as claimed in claim 1, wherein the base used is tetramethylurea.

6. A process as claimed in claim 1 or 5, wherein the base is simultaneously used as a solvent.

7. A process as claimed in claim 1, wherein the reaction component water or alcohol is simultaneously used as a solvent.

8. A process as claimed in claim 1, wherein the carboxylation is performed at from 20 to 200°C.

9. Saccharincarboxylic acids or -carboxylic acid esters of the formula I 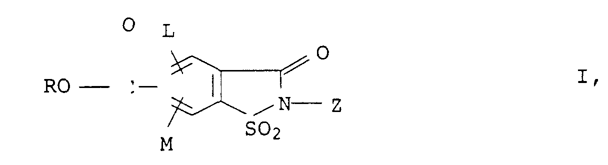 where the substituent have the following meanings:
L and M are hydrogen or C₁-C₄-alkyl,
Z is hydrogen or C₁-C₄-alkyl,
R is H or C₁-C₆-alkyl,
with the proviso that Z is not methyl or hydrogen or an alkali metal or silver cation if L and M are hydrogen, and further excluding 5-carboxy-7-methylsaccharin.

10. A herbicidal composition containing a compound Ia as claimed in claim 9 and customary inert carriers.

11. A method of controlling undesired plant growth, which comprises allowing a herbicidally active amount of a saccharincarboxylic acid or of a -carboxylic acid ester of the formula Ia 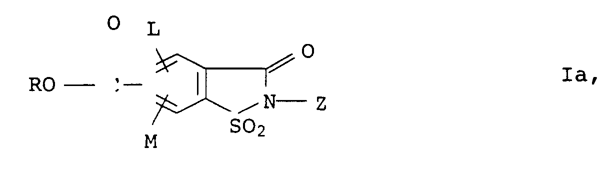 where the substituent have the following meanings:
L and M are hydrogen or C₁-C₄-alkyl,
Z is hydrogen or C₁-C₄-alkyl,
R is H or C₁-C₆-alkyl,
or an environmentally tolerable salt of the compounds Ia to act on the plants or their habitat.

## Revendications

1. Procédé pour la préparation d'acides saccharinecarboxyliques et d'esters d'acides saccharinecarboxyliques de formule I, 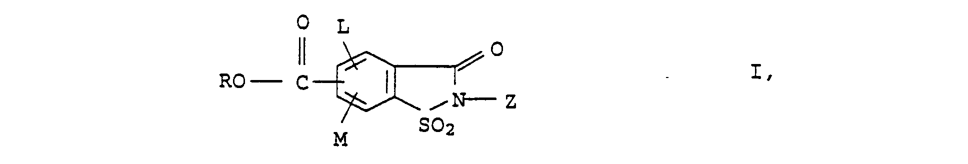 dans laquelle les substituants ont la signification suivante:
L, M hydrogène, alkyle en C₁-C₄;
Z hydrogène, alkyle en C₁-C₄;
R H, alkyle en C₁-C₆,
**caractérisé par** le fait qu'on fait réagir des dérivés de saccharine bromo- ou iodosubstitués correspondants de formule II 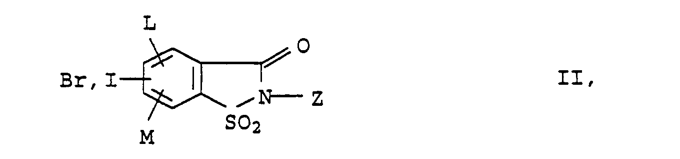 dans laquelle L, M et Z ont la signification susmentionnée, ou dans le cas où Z ≠ H des composés de formule III 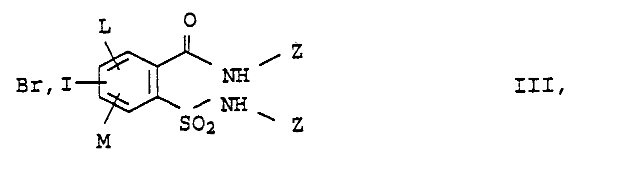 en présence d'un catalyseur à métal de transition palladium, nickel, cobalt ou rhodium et d'une base avec du monoxyde de carbone et de l'eau ou un alcool en C₁-C₆, sous pression élevée.

2. Procédé selon la revendication 1, **caractérisé par** le fait qu'on utilise des catalyseurs au palladium classiques pour des réactions de carbonylation.

3. Procédé selon la revendication 1, **caractérisé par** le fait qu'on effectue la carbonylation en présence de catalyseurs au palladium, qui contiennent des ligands de phosphine à base de phosphine tertiaire.

4. Procédé selon la revendication 1, **caractérisé par** le fait qu'on utilise comme base des amines tertiaires ou des dérivés d'urée tétraalkyl-substitués.

5. Procédé selon la revendication 1, **caractérisé par** le fait qu'on utilise comme base de la tétraméthylurée.

6. Procédé selon les revendications 1 et 5, **caractérisé par** le fait qu'on utilise la base simultanément comme solvant.

7. Procédé selon la revendication 1, **caractérisé par** le fait qu'on utilise le partenaire réactionnel eau ou alcool simultanément comme solvant.

8. Procédé selon la revendication 1, **caractérisé par** le fait qu'on effectue la carboxylation à une température de 20 à 200°C.

9. Acides saccharinecarboxyliques ou esters d'acides saccharinecarboxyliques de formule I 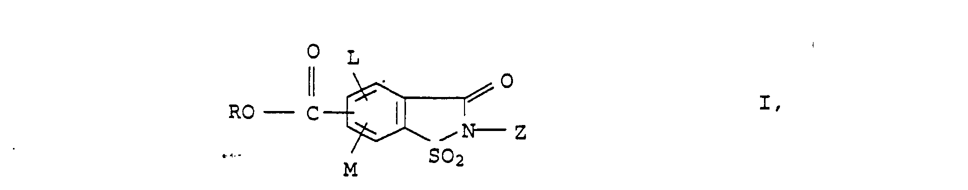 dans laquelle les substituants ont la signification suivante:
L, M hydrogène, alkyle en C₁-C₄,
Z hydrogène, alkyle en C₁-C₄,
R H, alkyle en C₁-C₆,
à l'exception de ce que Z ne désigne par un méthyle ou l'hydrogène ou un cation de métal alcalin ou d'argent, lorsque L et M représentent l'hydrogène, et également à l'exception de la 5-carboxy-7-méthylsaccharine.

10. Produit herbicide, contenant les composés Ia selon la revendication 9 et des supports inertes classiques.

11. Procédé pour la lutte contre la croissance parasite des plantes, **caractérisé par** le fait qu'on laisse agir une quantité efficace du point de vue herbicide d'un acide saccharinecarboxylique ou d'un ester d'acide saccharinecarboxylique de formule Ia 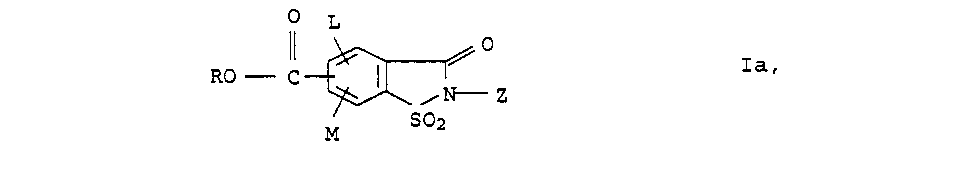 dans laquelle les substituants ont la signification suivante:
L, M hydrogène, alkyle en C₁-C₄,
Z hydrogène, alkyle en C₁-C₄,
R H, alkyle en C₁-C₆,
ou un sel acceptable pour l'environnement des composés Ia, sur les plantes ou leur biotope.
